# EUROPEAN PATENT APPLICATION

(11) **EP 2 583 940 A1**
(43) Date of publication of application: **24.04.2013**
(21) Application number: 11186177.9
(22) Date of filing: 21.10.2011
(51) Int. Cl.: C01B 31/02, C01B 31/04, C09B 61/00, C09B 67/00, A61K 9/10, A61K 47/32

(54) **Stabilization of carbon nanomaterials and hydrophobic compounds by copolymers**

(71) Applicant: Max-Planck-Gesellschaft zur Förderung der Wissenschaften e.V., 80539 München (DE)
(72) Inventor: Soll, Sebastian, 12157 Berlin (DE); Yuan, Jiayin, Dr., 14109 Berlin (DE); Antonietti, Markus, Prof.Dr., 14558 OT Nuthetal / Rehbrücke (DE)
(74) Representative: HOFFMANN EITLE

(57) **Abstract**

The present invention is concerned with dispersions of hydrophobic compounds and/or carbon nanomaterial, in particular carbon nanotubes (CNTs) such as single-walled carbon nanotubes (SWCNTs) and multi-walled carbon nanotubes (MWCNTs), and graphenes, comprising as a dispersing agent a copolymer comprising repeating units derived from ionic liquid (IL) monomer and repeating units derived from hydrophilic non-ionic vinyl monomer. According to further aspects, the present invention pertains to a method of dispersing hydrophobic compounds and/or carbon nanomaterial in a dispersion medium in the presence of such copolymers, the use of such copolymers for exfoliating carbon nanostructures, such as carbon nanotubes or graphene, as well as to specific copolymers *per se*. The dispersions of the invention, *e.g*. waterborne dispersions, are highly stable and can contain high concentrations of carbon nanomaterial and/or hydrophobic compounds.

## Description

### Field of the Invention

The present invention relates to, preferably waterborne, dispersions of hydrophobic compounds and/or carbon nanomaterial, in particular carbon nanotubes (CNTs) such as single-walled carbon nanotubes (SWCNTs) and multi-walled carbon nanotubes (MWCNTs), and graphenes, comprising as a dispersing agent a copolymer comprising repeating units derived from ionic liquid (IL) monomer and repeating units derived from hydrophilic non-ionic vinyl monomer. According to further aspects, the present invention is concerned with a method of dispersing hydrophobic compounds and/or carbon nanomaterial in a dispersion medium in the presence of such copolymers, the use of such copolymers for exfoliating carbon nanostructures, such as carbon nanotubes or graphene, as well as to specific copolymers *per se.*

### Background of the Invention

Processing of hydrophobic materials in aqueous solution is one of the major factors that govern their industrial production and practical application. Among them, carbon nanomaterial, and hydrophobic organic molecules are of special interests, as their aqueous dispersions constitute products of a wide range of technologically important applications, including nanoelectronics, drug delivery, composite devices of different types, and optical sensors. Waterborne dispersions of organic dyes and pharmaceutical molecules are attractive food ingredients or drugs due to an improved appearance of the comestible and a higher bioavailability of the dispersed compound (Nakajima et al., J. Agric. Food Chem., 2007, 55, 6754-6760). Water-based carbon dispersions are found in cosmetics (Mascara, eye lash extender), as inks and toners, or for general colour purposes, just to name a few.

Carbon nanostructures, such as carbon nanotubes (CNTs), graphene and fullerene, show extraordinary electric, mechanical, optical and chemical properties, which are of huge potential. Because of van der Waals attractions and hydrophobic interactions, they agglomerate readily into bundles. This is the apparent obstacle to fully utilize their potential. The manufacture of waterborne dispersions of carbon nanomaterials still remains a challenge, even in spite of recent progress in this field.

In general, two types of stabilization techniques can be distinguished, namely covalently or physically. The former is based on surface modifications that covalently modify the carbon nanostructures to make them stable in water. Physical methods are dealing with blending or mixing of the carbon nanostructures with another phase or with a stabilizer. The later is advantageous over the former, as it affects less or not at all the original unique properties of the carbon nanomaterial.

Surfactants or polymers have frequently been used to stabilize CNTs in water. The review article by C.-Y. Hu et al. in J. Chin. Chem. Soc., 2009, 56, 234-239 provides an overview of the non-covalent functionalization of CNTs with surfactants and polymers. In addition, WO 02/076888 A1 and DE 10 2006 055 106 A1 can be mentioned. Grunlan and co-workers reported the stabilization of CNTs in water with pyrene-functionalized poly(*N*-isoprapylacrylamide) and pyrene-functionalized poly(N-cyclopropylacrylamide) (K. C. Etika et al., Macromol. Rapid. Commun., 2010, 31, 1368-1373; K. C. Etika et al., J. Am. Chem. Soc., 2009, 131, 13598-13599). In WO 2010/102759 A1, a method of dispersing graphite-like nanoparticles using dispersing agents consisting of block copolymers is described. At least one block of the block copolymers carries aromatic side chains.

Recently, poly(ionic liquid)s (PILs), *i.e*. polymers obtained via the polymerization of ionic liquid (IL) monomers, have been shown to have a stabilizing effect for carbon nanostructures. Fukushima and Aida and co-workers found that CNTs, when mixed with imidazolium-based ILs and PILs, formed gels after being ground and can as such be seen as a new class of dispersants for CNTs (T. Fukushima et al., Science 2003, 300, 2072-2074; T. Fukushima, T. and T. Aida, Chem. Eur. J. 2007, 13, 5048-5058). T. Sekitani et al. in Science 2008, 321, 1468-1472 report that they succeeded by using an IL of 1-butyl-3-methylimidazolium bis (trifluoromethanesulfonyl) imide to uniformly disperse SWNTs as chemically stable dopants in a vinylidene fluoride-hexafluoropropylene copolymer matrix to form a composite film.

R. Marcilla et al. in Small, 2006, 2, 507-512 employed the PIL poly(1-vinyl-3-ethylimidazolium bromide) as a dispersant to stabilize SWCNTs in aqueous solutions.

Using polyd-vinyl-3-ethylimidazolium salts as PILs T. Kim et al. (ACS Nano, 2010, 4, 1612-1618) stabilized isolated graphene sheets and provided a functionality for transferring graphene sheets between the aqueous and organic phases. A. A. Green and M. C. Hersam in J. Phys. Chem. Lett., 2010, 1, 544-549 provide an overview of methods for producing monodisperse graphene dispersions, and the wet chemistry of graphene is summarized by M. Englert et al. in The Electrochemcial Society Interfacs, Spring 2011 edition, 53-56.

The potential of PILs as dispersants to stabilize nanomaterials in aqueous and organic solution has been summarized by J. Yuan and M. Antonietti in Polymer, 2011, 52, 1469-1482.

Furthermore Antonietti et al. in Appl. Mat. Int., 2010, 2, 3, 649-653 reported a nanolatex copolymer (25-30 nm) of an imidazolium bromide acrylate that provided stable waterborne dispersions of SWCNTs. Specifically, the nanolatex was prepared by microemulsion polymerization in the ternary system of water, 1-(2-acryloyloxyundecyl)-3-methylimidazolium bromide (IL-Br) and methylmethacrylate (MMA). The nanolatex copolymer comprises high proportions of IL-Br. The present inventors found that spherical latex particles are formed, which are stable and useful for stabilizing CNTs only when larger proportions of IL-Br are present in the copolymer. The same and similar imidazolium-based ionic liquids were used by F. Yan and J. Texter to stabilize polymerizable microemulsions useful for producing polymer nanoparticles, gels and open-cell porous materials (Chem. Commun., 2006, 2696-2698; Angew. Chem. Int. Ed. 2007, 46, 2440-2443).

PILs and copolymers comprising high proportions of ILs as dispersants are however less attractive for large-scale applications, as they are quite expensive.

In view of the above, the present inventors were faced with the object of providing more economical dispersants being excellently suited for dispersing hydrophobic materials such as carbon nanomaterial and/or hydrophobic compounds.

### Summary of the Invention

The present invention is based on the unexpected finding that copolymers comprising repeating units derived from ionic liquid (IL) monomer and repeating units derived from hydrophilic non-ionic vinyl monomer (in short "copolymer(s) of the invention"), even when they comprise a low fraction of the IL monomer-derived repeating units, are most effective stabilizers for carbon nanomaterials and hydrophobic compounds for instance in polar solvents, such as aqueous solutions. It is worth noting that the non-ionic monomer units in the copolymers of the invention are not able to stabilize carbon nanostructures for example in water, due to only weak interactions. Nevertheless, the copolymers of the invention proved to be more efficient stabilizers than PIL homopolymers, which consist exclusively of IL monomer units.

Since the amount of expensive IL monomer-derived repeating units can be reduced in the copolymers of the invention beyond what is feasible in the systems of the prior art, an additional benefit of the present invention is the cost effectiveness. This is because the copolymer stabilizers of the invention can consist chiefly of hydrophilic non-ionic monomer-derived repeating units. Moreover, the hydrophilic non-ionic vinyl monomer can be selected from a broad range of industrially available monomers.

Accordingly, the present invention, according to Claim 1, provides a dispersion of carbon nanomaterial and/or hydrophobic compounds, which dispersion comprises a copolymer comprising repeating units derived from ionic liquid monomer(s) and repeating units derived from hydrophilic non-ionic vinyl monomer(s). Moreover, a method of dispersing carbon nanostructures and/or hydrophobic compounds in a dispersion medium is provided by the present invention in independent Claim 14, which dispersing method comprises the step of dispersing the carbon nanostructures and/or hydrophobic compounds in the dispersion medium by introducing an amount of energy sufficient for the dispersion, preferably by ultrasonic treatment, in the presence of a copolymer of the invention as a dispersing agent. Furthermore, the present invention in independent Claim 15 envisages the use of a copolymer of the invention for exfoliating carbon nanostructures, such as carbon nanotubes or graphene.

According to another aspect of the present invention as defined in independent Claim 12, a copolymer comprising repeating units derived from IL monomer(s) and repeating units derived from hydrophilic non-ionic vinyl monomer(s), is provided, wherein the molar ratio of the repeating units derived from the hydrophilic non-ionic vinyl monomer(s) and the repeating units derived from the ionic liquid monomer(s) is in the range of from 4:1 to 200:1 and preferably in the range of from 5:1 to 100:1, and the hydrophilic non-ionic vinyl monomer(s) is/are an acrylamide or a styrenic monomer. This particular copolymer proved to be a highly effective dispersing agent or stabilizer in the dispersions of the invention.

Preferred embodiments of the present invention are subject of the dependent claims.

### Brief Description of the Drawings

Fig. 1 shows a representative ¹H-NMR spectrum of the copolymer poly(NIPAM-co-EVImBr) (EVImBr content of 7.6 mol. %) in D₂O. In the present specification "NIPAM" stands for *N*-isopropylacrylamide and "EVImBr" for 1-ethyl-3-vinylimidazolium bromide.
Fig. 2 shows photographs of aqueous dispersions of MWCNTs in water with PNIPAM homopolymer (left) and poly(NIPAM-co-EVImBr) (EVImBr content of 7.6 mol. %) copolymer (right) as stabilizer 24 h after sonication treatment.
Fig. 3 shows representative TEM (A and B) and Cryo-TEM (C and D) images of dispersed MWCNTs (0.02 wt%) in aqueous solution using poly(NIPAM-*co*-EVImBr) (EVImBr content of 7.6 mol. %) (0.4 wt%) as stabilizer. The inset in Fig. 3A is a photograph of the formed stable dispersion of MWCNTs taken 24 h after sonication treatment.
Fig. 4 presents plots of transmission vs. temperature of aqueous solutions (10 g/L) of poly(NIPAM-co-EVImBr) with 7.6 mol. % of EVImBr at different concentrations of KBr (A), and with different EVImBr monomer unit content and a constant concentration of KBr *(C_{KBr}* = 0.015 M) (B).
Fig. 5 provides a schematic illustration of temperature and ionic strength responsive CNT dispersion by using a copolymer stabilizer. The symbol "Δ" denotes heating.
Fig. 6 illustrates the destabilization of MWCNTs in aqueous solution at different temperatures. Here the same copolymer stabilizer: poly(NIPAM-co-EVImBr) with 7.6 mol.% of EVImBr was used for all vials. The concentration of KBr from left to right vials was 1, 0.1, 0.05 and 0 M.
Fig. 7 illustrates the destabilization of MWCNTs in aqueous solution at a specific temperature. Left: samples at temperature lower than 38°C; right samples at 38°C. In each photograph, in the left vial, the copolymer stabilizer poly(NIPAM-ca-EVImBr) contained 7.6 mol. % of EVImBr, *C_{KBr}* ~ 0.1 M; in the right one, it contained 2.2 mol. % of EVImBr, *C_{KBr} ~* 0.013 M.
Fig. 8 is a graph showing the optical density upon dispersing CNTs in water using ultrasonic treatment as a function of time when using as a dispersing agent a copolymer of the invention in comparison to a PIL.

### Detailed Description of the Invention

The dispersion of carbon nanomaterial and/or hydrophobic compounds comprising a copolymer, wherein the copolymer comprises repeating units derived from ionic liquid monomer(s) and repeating units derived from hydrophilic non-ionic vinyl monomer(s), is sometimes termed "dispersion of the invention" herein. Moreover the copolymer present in the dispersion of the invention is occasionally referred to in this specification as "copolymer of the invention".

In the present specification, the term "dispersion" is used in its usual meaning, *i.e*. as referring to a system consisting of several phases, one of which is a continuous phase (termed "dispersion medium") and at least one further phase of which is finely distributed (termed "dispersed phase"). Accordingly, in the dispersion of the present invention, the carbon nanomaterial and/or hydrophobic compounds represent the dispersed phase. Moreover, the copolymer of the invention serves as a dispersing agent, *i.e*. a substance facilitating the dispersion of the carbon nanomaterial and/or hydrophobic compounds in the dispersion medium. As the copolymer of the invention also has a stabilizing effect on the dispersion, it is sometimes also referred to as a "stabilizer" in the present specification.

According to a preferred embodiment, the dispersion of the invention is a dispersion of carbon nanomaterial. The term "carbon nanomaterial" as used herein, means carbon-based material, *i.e*. material comprising at least 90 wt%, preferably at least 95 wt% carbon, with morphological features on the nanoscale. That is, the material has a structure, at least one dimension of which is in the range of 0.1 to 100 nm, especially 1 to 100 nm. So the carbon nanomaterial for use in the dispersion of the invention have a nanostructure, *i.e*. a structure having at least one dimension in the 0.1 to 100 nm, especially 1 to 100 nm size range.

The carbon nanomaterial that can constitute the dispersed phase of the dispersion of the present invention is preferably selected from the group consisting of carbon nanotubes, fullerenes, graphene and soot.

Carbon nanotubes (CNTs) are allotropes of carbon with a cylindrical nanostructure. They can have a diameter of 0.1 to 100 nm. Their length-to-diameter ratio can be more than 10⁶. Carbon nanotubes are categorized as single-walled nanotubes (SWNTs) and multi-walled nanotubes (MWNTs). CNTs can be produced by arc discharge between carbon electrodes, laser ablation, *e.g*. of graphite, and CVD processes, *e.g*. by catalytic decomposition of hydrocarbons (CCVD). CNTs are commercially available, for instance from Aldrich and from Bayer MaterialScience AG (Baytubes^{®}).

The carbon nanomaterial for use in the dispersion of the present invention can also be fullerenes, for instance C₆₀, C₇₀, C₇₆, C₈₀, C₈₂, C₈₄, C₈₆, C₉₀ and C₉₄, in particular C₆₀, C₇₀ and C₇₂, some of which are commercially available.

According to another embodiment of the dispersions of the present invention, the carbon nanomaterial is graphene. Graphene is another allotrope of carbon with a two-dimensional structure, in which each carbon atom is surrounded by three other carbon atoms, so that a honeycomb crystal lattice is formed. Consequently, graphene is a flat monolayer of carbon atoms tightly packed into a two-dimensional honeycomb lattice. Graphene can be regarded as a single graphite layer. The synthesis of graphene is described by T.J. Booth et al. in Nano Letters, 2008, 8, 2442-2446, and a review of the synthesis, properties and applications of graphene and graphene oxide is given by Y. Zhu et al. in Adv. Mater, 2010, 22, 3906-3924. Moreover, graphenes are commercially available e.g. from Graphene Industries.

Finally, the carbon nanomaterial for use in the dispersion of the present invention can be soot, which is understood herein as referring to nanoparticles, *i.e*. particles typically having a diameter of 1 to 100 nm, resulting from the incomplete combustion of a hydrocarbon. S.C. Graham et al. in Proc. R. Soc. Lond. A., 1975, 344, 259-285, deal with the synthesis of soot. Moreover, soot can be obtained from Evonik Industries AG and Lehmann & Voss & Co. as suppliers.

In another embodiment of the present invention, hydrophobic compounds form the dispersed phase in the dispersion of the invention. The hydrophobic compounds to be dispersed in the dispersion of the invention preferably have solubility in water of < 20 g/L at 20°C. Stated in more general terms, the hydrophobic compounds to be dispersed in the dispersion medium to give the dispersion of the invention preferably have solubility in the dispersion medium, which preferably is a polar solvent such as water, of < 20 g/L, and more preferably < 1 g/L at 20°C. For instance, the dispersion of the invention can be a dispersion of hydrophobic compounds having a solubility in water at 20°C of less than 1 g/L, which dispersion comprises the copolymer of the invention as a dispersing agent, and water as a dispersion medium, *i.e*. it is a waterborne dispersion of these particular hydrophobic compounds.

The hydrophobic compounds for use in the present invention are preferably hydrophobic organic compounds. Examples are dyes and pharmaceutically active components ("drugs"). Without limitation, examples are β-carotene, vitamins A, D, E and K, itraconazole, tacrolimus, cyclosporine A, carbamazepine, fentanyl and amphotericin B.

Taking account of the above, according to a preferred embodiment, the dispersion of the present invention is a dispersion of CNTs comprising a copolymer of the invention as a dispersing agent, and a polar solvent as a dispersion medium.

According to an alternative embodiment, the dispersion of the invention is a dispersion of hydrophobic compounds comprising as a dispersing agent the copolymer of the invention and a polar solvent as a dispersion medium, in which the hydrophobic compounds would be insoluble in the given concentrations without adding the copolymer of the invention.

The copolymer of the invention comprises, preferably consists of, repeating units derived from ionic liquid (IL) monomer(s) and repeating units derived from hydrophilic non-ionic vinyl monomer(s).

As used herein, "ionic liquid monomer(s)" are monomers that are composed of cations and anions which melt below 100°C. Preferably, the ionic liquid (IL) monomers comprise a cationic group selected from the group consisting of imidazolium, pyridinium, alkylammonium, alkylphosphonium, pyrrolidinium and guanidinium, and/or an anion selected from the group consisting of halides, NO₃⁻, BF₄⁻, [BR¹R²R³R⁴]⁻, PF₆⁻, [(CF₃SO₂)₂N]⁻, [CF₃SO₃]⁻, [CF₃CO₂]⁻ and [CH₃CO₂]⁻, wherein R¹, R², R³ and R⁴ each represent alkyl or aryl.

In a preferred embodiment, the IL monomers comprise as a cationic group an imidazolium group, *i.e*. they are imidazolium-based IL monomers.

According to a preferred embodiment, the IL monomer is represented by the following formula (I): 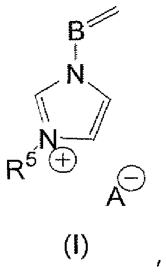 wherein R⁵ is alkyl, aryl or arylalkyl, in particular C₁₋₆ alkyl, C₆₋₁₄ aryl or C₆₋₁₄ aryl-C₁₋₆ alkyl; B represents a linking group, such as an alkylene group, for instance a C₁₋₆ alkylene group; and A⁻ represents an anion. The anion A⁻can for instance be selected from the group consisting of halides, NO₃⁻, BF₄⁻, [BR¹R²R³R⁴]⁻, PF₆⁻, [(CF₃SO₂)₂N]⁻, [CF₃SO₃]⁻, [CF₃CO₂]⁻ and [CH₃CO₂]⁻, wherein R¹, R², R³ and R⁴ each represent alkyl or aryl, for instance C₁₋₆ alkyl or C₆₋₁₄ aryl. In a preferred embodiment, R⁵ is C₁₋₆ alkyl and B is CH in formula (I).

Furthermore, the copolymer of the invention comprises repeating units derived from hydrophilic non-ionic vinyl monomer(s). That means the monomer does not have a charge and comprises a vinyl group (-CH=CH₂). Monomers are defined herein as hydrophilic when they have solubility in water of > 20 g/L at 20°C. As such, they are distinguished from methylmethacrylate (MMA) that has been used as a comonomer in the prior art. The hydrophilic non-ionic vinyl monomer for preparing the copolymer of the invention is not specifically limited in kind as long as it is hydrophilic in the above sense. For instance, it can be selected from the group consisting of acrylamides, acrylates, methacrylates (other than the parent compound MMA, *i.e*. carrying suitable hydrophilic substituents) and styrenic monomers. As meant herein, "styrenic monomer" refers to a compound having a styrene core with suitable hydrophobic substituents at the benzene ring, which compound is polymerizable through its vinyl group. According to a preferred embodiment, the hydrophilic non-ionic vinyl monomer is an acryl amide. N-isopropyl acryl amide (NIPAM) is particularly preferred as it allows stimuli responsive dispersions to be prepared (see Examples 4 and 5).

According to a particularly preferred embodiment, the copolymer of the invention is a copolymer derived from an N-substituted acrylamide (such as NIPAM) and a salt of 1-C₁₋₆ alkyl-3-vinylimidazolium salt (such as 1-ethyl-3-vinylimidazolium bromide, EVImBr).

In the present specification, the expression "a copolymer comprising repeating units derived from (...) monomer" is construed to mean that the monomer in question will be incorporated upon polymerization as a repeating unit in the copolymer. Thereby the polymerizable group in the monomer(s), e.g. the unsaturated vinyl group, will be converted to a saturated C-C bond during the polymerization to give the copolymer.

The copolymer of the invention is preferably a random copolymer, especially a copolymer obtainable by free radical copolymerization of the ionic liquid monomer(s), the hydrophilic non-ionic vinyl monomer(s) and optionally further monomer(s). According to a preferred embodiment, the free radical copolymerization is performed in a solvent selected from the group consisting of methanol, ethanol, water, DMF (*i.e*. dimethylformamide) and DMSO (*i.e*. dimethylsulfoxide).

When the copolymers of the invention are used as dispersing agents, long-term stable dispersions of carbon nanomaterials and hydrophobic compounds, *e.g*. in polar solvents such as water as dispersion media, can be obtained. The polar solvents can be ethers, alcohols, carboxylic esters, lactones, aldehydes and ketones. According to a preferred embodiment, the dispersion medium is water. In this case, the dispersion is a waterborne dispersion, or in different terms an aqueous dispersion. In the present invention, concentrations of CNTs as high as 1.4 wt% could be stably dispersed in water. To the best of the inventors' knowledge, this is the waterborne stable CNT dispersion with the highest CNT concentration ever reported in the literature.

The copolymers of the invention are highly efficient dispersing agents, *i.e*. stabilizers, even when there is only a low proportion of repeating units derived from IL monomers contained therein. For instance, the ratio of the repeating units derived from the hydrophilic non-ionic vinyl monomer and the repeating units derived from the IL monomer may be in the range of 4:1 to 200:1, and preferably they are in the range of from 5:1 to 100:1. Such copolymer stabilizers are highly attractive in terms of cost.

In the dispersions of the invention, the copolymer of the invention is contained as a dispersing agent in usual amounts known to the skilled person, preferably in a concentration, in terms of the overall weight of the dispersion, of 0.01 to 10% by weight, more preferably 0.1 to 5% by weight.

In the method of dispersing carbon nanomaterial and/or hydrophobic compounds in a dipersion medium in accordance with the present invention, an amount of energy sufficient for the dispersion is introduced in the presence of a copolymer of the invention. The necessary energy can be introduced for instance by agitating, stirring and ultrasonic treatment, with ultrasonic treatment (sometimes denoted "sonication", herein) being preferred.

Carbon nanomaterials, in particular CNTs and graphene tend to stick together, thus forming agglomerates. Through the use of the copolymers of the invention, these agglomerates can be disintegrated to give separate nanostructures, *e.g*. individual CNTs and graphene monolayers, which remain stable for time periods of at least days. The above de-agglomeration of nanostructures, such as CNTs or graphene, is denoted as "exfoliating" in this specification.

Another benefit is that the copolymers of the invention may contain, apart from the ILL monomer-derived repeating unit, a water-soluble non-ionic comonomer that provides additional functionality and values to the aqueous dispersions of carbon nanomaterials and hydrophobic organic compounds. As one example, a copolymer of NIPAM and an IL monomer can be applied as a smart stabilizer that cannot only disperse carbon nanosmaterials or hydrophobic compounds, but also respond to external environmental changes, which might be very useful for biological and nanomedical applications.

The present invention is further described by the following examples, which must of course not be construed in a limiting sense.

### Examples

### Preparation Example

### Materials

1-vinylimidazole (Aldrich 99%) and bromoethane (Aldrich 98%) were used as received without purification, AIBN, *i.e*. 2,2'-azobis(2-methylpropionitrile) (Aldrich 98%), was recrystallized from hexane, *N*-isopropylacrylamide (Acros 99%) was recrystallized from hexane/toluene (3:1), single-walled carbon nanotubes (SWCNTs) (Aldrich, 0.7-1.1 nm in diameter, 0.3-2.3 µm in length, 90% carbon content) and multi-walled carbon nanotubes (Baytubes^{®} C150P) were purified as described below. All solvents used were of analytical grade.

### Synthesis of ionic liquid monomer EVImBr

9.41 g (0.1 mol) of 1-vinylimidazole, 12.3 g (0.1 mol) of bromoethane and 30 mL of methanol were charged into a 100 mL reactor. The mixture was stirred at 60°C for 15 h. After cooling down, the reaction mixture was added dropwise into 1 L of diethyl ether. The white precipitate was filtered off and dried at room temperature until constant weight to give the target compound EVImBr, i.e. 1-ethyl-3-vinylimidazolium bromide.

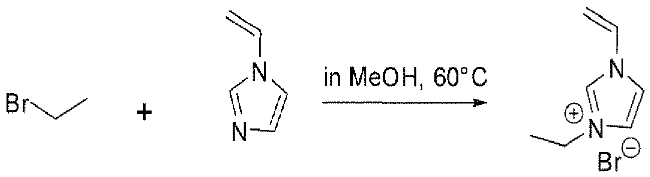

### Purification of CNTs

300 mg of carbon nanotubes (CNTs) were refluxed in 50 mL of 3M HNO₃ for 12 h in a 250 mL flask to remove the catalyst. After cooling down, the CNTs were separated by ultrazentrifugation, washed with excess of water several times and freeze-dried.

### Example 1: Synthesis of poly(NIPAM-co-EVImBr)

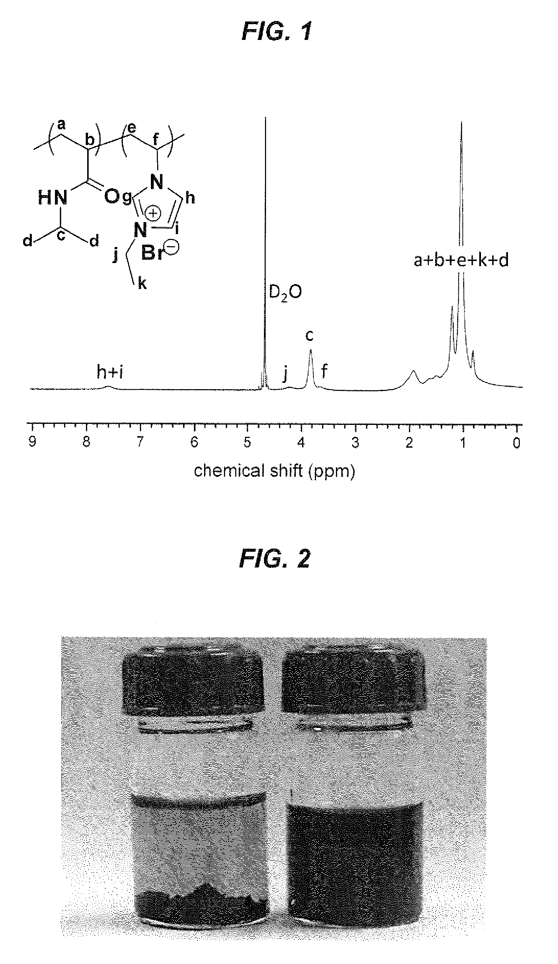

The copolymers with a molar content of EVImBr of 2.2, 4.3, 5.4, 7.6 and 12.8%, are prepared from the conventional free radical copolymerizations of NIPAM and EVImBr in various molar ratios (40:1, 20:1, 15:1, 10:1, and 5:1). In the following the procedure to prepare the copolymer with 7.6 mol% of EVImBr given as an example: 5.65 g (0.05 mol) of *N-*isopropylacrylamide (NIPAM), 1.01 g (0.005 mol) of 3-ethyl-1-vinylimidazolium bromide (EVImBr) as obtained above, 82 mg (0.5 mmol) of AIBN and 50 mL of methanol were added to a 100 mL flask. The mixture was sealed with a septum and deoxygenated by flushing with argon for 30 min. The flask was then stirred for 15 h in an oil bath whose temperature was kept at 60°C using a thermostat. After cooling down, the reaction mixture was added dropwise into 500 mL of diethyl ether. The white precipitate was filtered off and dried under vacuum until constant weight. The obtained copolymer was then re-dissolved in methanol and exhaustively dialyzed against de-ionized water. After freeze-drying, 5.8 g of the product poly(NIPAM-co-EVImBr) was obtained. A representative ¹H-NMR spectrum of the product is shown in Fig. 1. The copolymerization of NIPAM and EVImBr is illustrated in the following scheme.

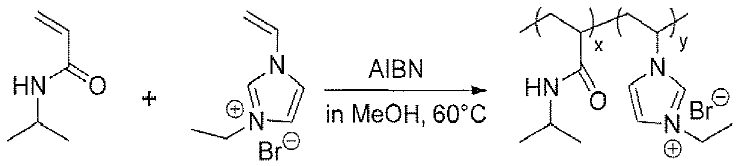

### Example 2: Dispersion of MWCNTs

In a representative dispersing procedure, 5 mg of MWCNTs and 100 mg of copolymer stabilizer poly(NIPAM-co-EVImBr) (EVImBr content of 7.6 mol. %) were mixed in 25 mL of water. The mixture was first subjected to mild sonication in an ultrasonic bath for 30 min, where a partial dissociation of carbon nanotubes started. Then a strong sonication (60% amplitude, Branson sonifier W450 Digital, mode: 2s on and 8s off) was applied to the aqueous mixture for 1 h to disperse the MWCNTs in the solution. The temperature of the dispersion was controlled within the range of 10-20 °C using a thermostat during the sonication process.

Figure 2 shows a photograph taken 24 h after dispersing MWCNTs in aqueous solutions using PNIPAM homopolymer (left) and poly(NIPAM-*co*-EVImBr) copolymer (right) as stabilizer. It is clearly seen that in the presence of a homopolymer stabilizer of PNIPAM, MWCNTs precipitated down to the bottom when ultrasound was switched off. On the contrary, a stable aqueous dispersion of MWCNTs was obtained in the presence of a copolymer stabilizer of poly(NIPAM-*co*-EVImBr). This evidently proves that the stabilizing of MWCNTs in aqueous solution by a copolymer of poly(NIPAM-co-EVImBr) is successful and exclusively stems from the stabilizing effect of the IL monomer units in the copolymer.

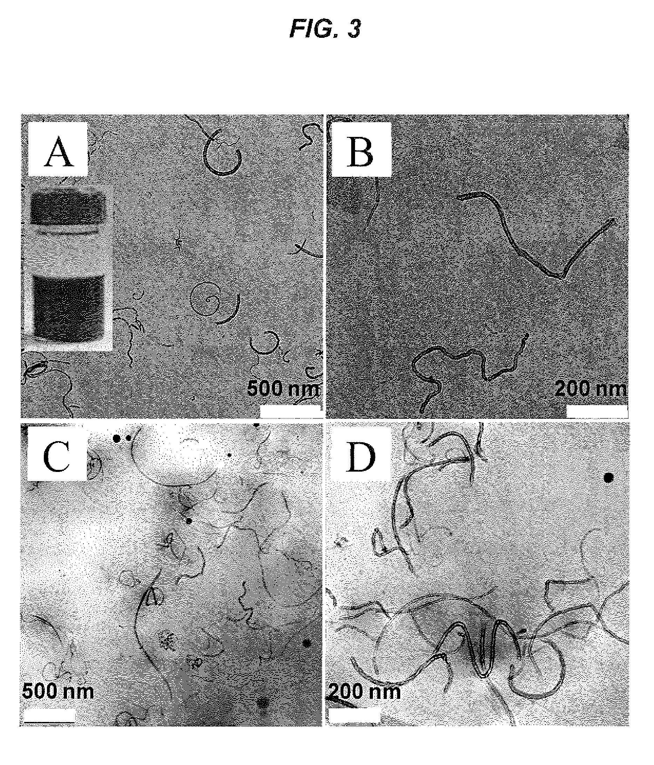

Transmission electron microscopy (TEM) and Cryo-TEM characterization of the aqueous dispersion of MWCNTs stabilized by the copolymer of the invention was conducted. Figure 3 shows two representative TEM images (Fig. 3A and 3B) of a MWCNT dispersion sample. It is seen that large sized-agglomerates of MWCNTs are essentially absent. MWCNTs, independent of their length and diameter, are well disentangled. Cryo-TEM measurements (Fig. 3C and 3D) demonstrate that in a real solution state the MWCNTs are indeed well-dispersed even at a high local concentration. Thus, the presence of the copolymer of the present invention as a stabilizer allowed exfoliation of the hydrophobic MWCNTs via sonication and immediate formation of a homogeneous aqueous dispersion.

### Example 3: Dispersion of β-carotene

Dispersions of β-carotene were prepared by adding 5 mg of β-carotene and 100 mg of poly(NIPAM-*co*-EVImBr) (EVImBr content of 7.6 mol. %) copolymer in 25 mL of water. The mixture was subjected to mild ultrasonic treatment in an ultrasonic bath for 30 min, followed by a strong sonication (60% amplitude, Branson sonifier W450 Digital, mode: 2s on and 8s off) for lh to disperse the β-carotene in the solution. A homogeneous dispersion of β-carotene in the aqueous phase instead of a phase-separated mixture was obtained and remained stable 24 h after the sonication treatment and beyond. This proves that β-carotene forms stable aqueous dispersion in the presence of the copolymer of the present invention as a stabilizer assisted by ultrasonic treatment.

### Example 4: Stimuli responsiveness of copolymers

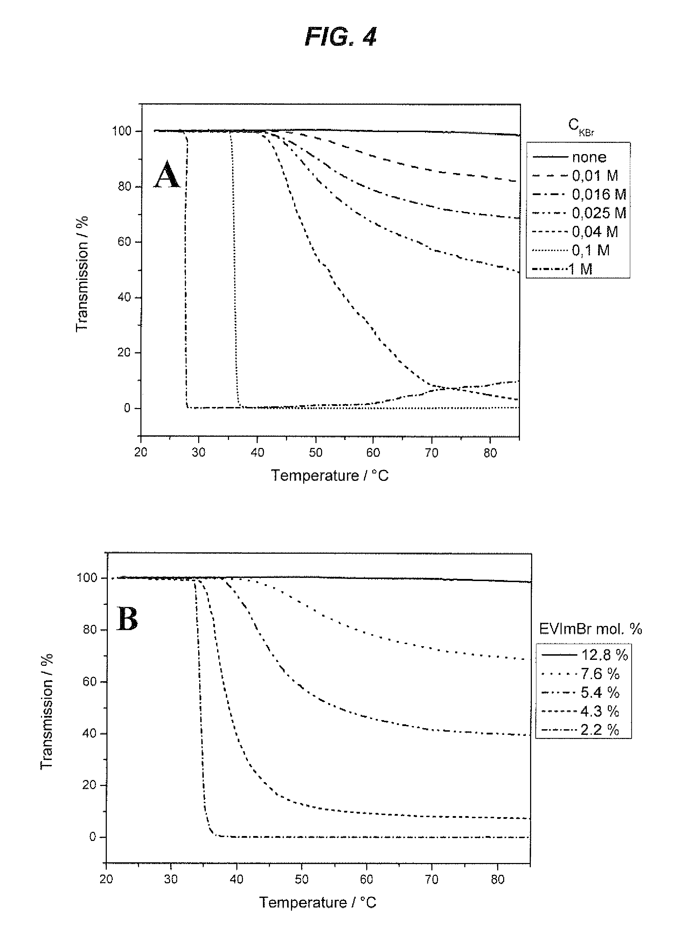

In the copolymer stabilizer which contains thermosensitive monomer units, for example, NIPAM, a double stimuli-responsive behavior of their aqueous solution against temperature and ionic strength can be observed. In a typical experiment, aqueous solutions of poly(NIPAM-*co*-EVImBr) (EVImBr content of 7.6 mol. %) at different concentrations of KBr were subjected to temperature dependent UV/Vis spectroscopy. Fig. 4A shows the turbidity curve of the poly(NTPAM-*co*-EVTmBr) copolymer at different concentrations of KBr salt (C_{KBr}). Without any salt (C_{KBR} = 0), the copolymers are stable even at 90°C. With increasing concentration of KBr, the cloud point (defined as 80% of the transmittance here) appears and shifted to lower temperature range. At a sufficient concentration of KBr, here at 0.04 M, the copolymer precipitated above 70°C. At even higher concentration of 0.1 M and 1 M, the copolymer precipitated at low temperature of 25-40°C. Thus, by changing the amount of added salt (KBr) the cloud point of the copolymer solution can be shifted. Apart from ionic strength, the chemical composition of the copolymers plays also an important role in determining the cloud point and stability of the copolymer solution. Fig. 4B shows the turbidity curve of the paly(NTPAM-co-EVImBr) copolymer at the same KBr concentration *(C_{KBr}* = 0.015 M) but at different ratios of NIPAM/IL monomer. It is clearly seen that at a constant concentration of KBr salt, the turbidity becomes more sensitive with increasing NIPAM content (up to 97.8 mol%). Indeed, the variation of molar ratio of NIPAM/ IL monomer results in a dramatic change of the transition temperature of the copolymer in aqueous solution.

### Example 5: Temperature- and salt-sensitive CNT dispersions

The stimuli-responsiveness of poly(NIPAM-*co*-EVImBr) copolymer (as studied in Example 4) can be employed to prepare temperature- and salt-sensitive carbon nanotube dispersions. This is because when both NIPAM and IL monomer units in the copolymer chains collapse, carbon nanotubes will be no longer solubilised and precipitate out from the aqueous solution.

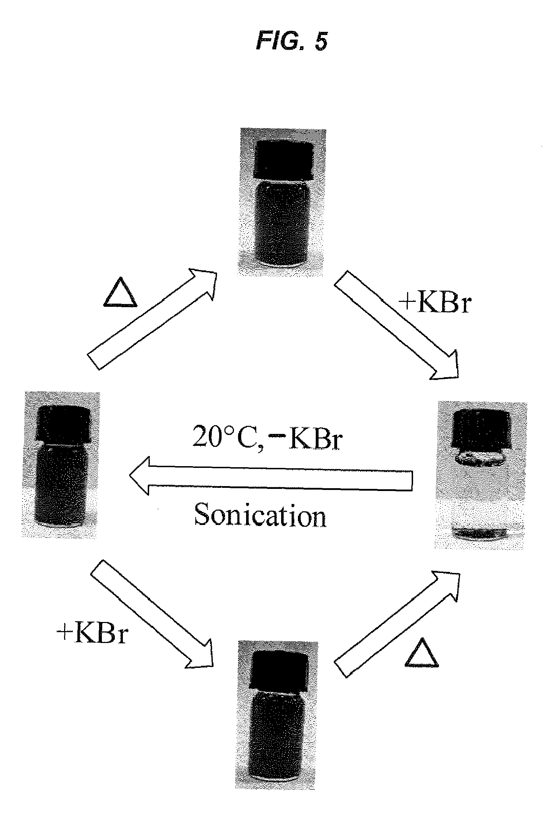

Figure 5 shows a scheme with photographs of the aqueous MWCNT dispersions at different environments. At room temperature, the MWCNTs are stabilized by the copolymer stabilizer (left photograph). By heating to 60°C (upper photograph) or by adding excessive KBr salt (lower photograph), the MWCNT dispersion remain stable. Only the combination of heating and adding salt together leads to a precipitation of the carbon nanotubes (right photograph). The precipitated carbon nanotubes can be redispersed by sonication at 20°C, if salt is removed (see central arrow).

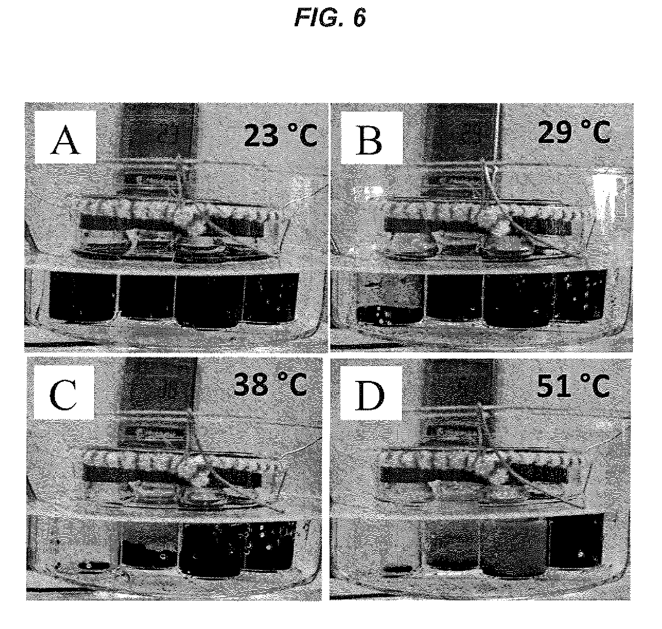

Figure 6 shows that it is practically possible to tune the specific temperature, when precipitation of carbon nanotubes should start in dependence on the amount of added salt. The 4 vials shown contain dispersions of MWCNTs in water with poly(NIPAM-co-EVImBr) (EVImBr content of 7.6 mol. %) and different concentrations of added KBr (from left to right: 1 M, 0.1 M, 0.05 M, none). Below 29°C (for example at 23 °C), all dispersions of carbon nanotubes are stable (Fig. 6A). When the temperature was increased to 29°C, the dispersion with 1M of KBr salt started to precipitate, while the rest remained unchanged (Fig. 6B). At a higher temperature of 38 °C, the dispersion with 0.1 M of KBr precipitated, too (Fig. 6C). At an even higher temperature of 51 °C, also the dispersion with 0.05 M of KBr precipitated (Fig. 6D). The dispersion of MWCNTs without any KBr salts stayed stable even at 90°C. This experiment illustrates the great benefit of using a copolymer comprising IL monomer units rather than a PIL homopolymer as stabilizer because it is possible to tune the stability of the dispersions by various means.

Example 6: Dispersing ability of copolymers of the invention in comparison to homopolymers

5 mg of MWCNT, 100 mg of polymer stabilizer (Poly(EVImBr) for comparison; and Poly(NIPAM-co-EVImBr) with 7.6 mol% EVImBr in accordance with the invention) and 25 mL of water were added in a 25 mL vial. At first the mixture was sonicated by a Bandelin Electronics (Berlin, Germany) Sonorex RK 100 ultrasonic bath for 60 s, where a partial dissociation of carbon nanotubes started. Then a Branson Digital Sonifier model W450D was used for more intensive sonication treatment of the aqueous mixture. Hereby a microtip (1/8" in diameter) was immersed in the dispersion and the sonifier was run at 10%, 30% and 60% of maximum amplitude for various time intervals (see table below).

| Dose time / s | Amplitude / % |
|---|---|
| 30 | 10 |
| 30 | 10 |
| 60 | 10 |
| 60 | 10 |
| 60 | 30 |
| 60 | 60 |
| 120 | 60 |
| 300 | 60 |

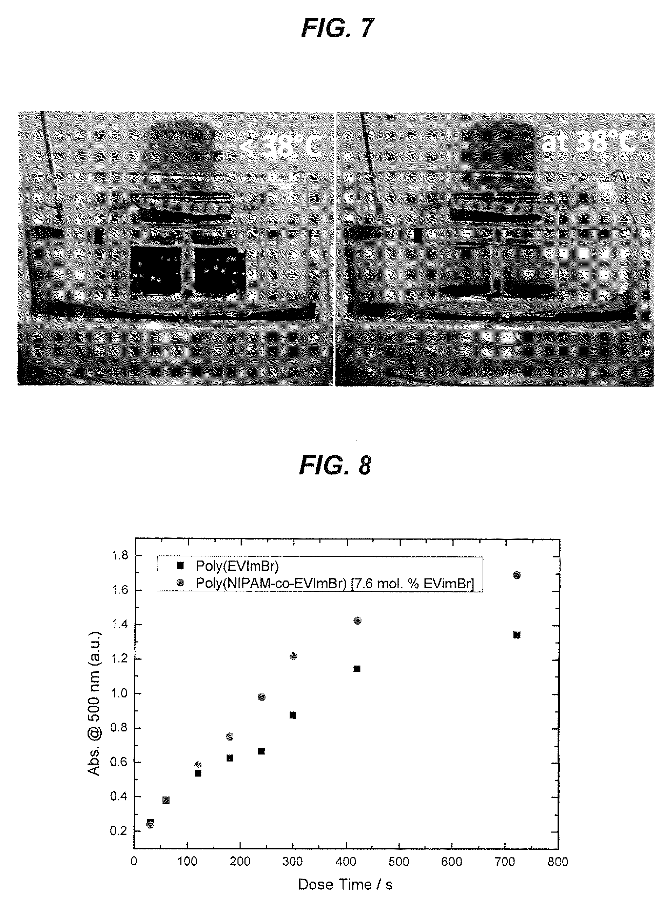

The dispersion was diluted fivefold and the optical density measured in a 1 cm cuvette. The results, *i.e*. the optical density as a function of time, are shown in Fig. 8. From the slope of the curves and the maximum optical density achieved, the quality of the dispersion can be assessed. It can be seen that the copolymers of the invention apart from being by far less expensive, are more efficient in dispersing CNTs in comparison to the homopolymers (PILs).

## Claims

1. Dispersion of carbon nanomaterial and/or hydrophobic compounds, the dispersion comprising a copolymer comprising repeating units derived from ionic liquid monomer and repeating units derived from hydrophilic non-ionic vinyl monomer.

2. The dispersion of Claim 1, which is a waterborne dispersion.

3. The dispersion of Claim 1 or 2, which is a dispersion of carbon nanomaterial, which is preferably selected from the group consisting of carbon nanotubes, fullerenes, graphene, and soot.

4. The dispersion of Claim 1 or 2, which is a dispersion of hydrophobic compounds, which are preferably selected from the group consisting of dyes or pharmaceutically active compounds.

5. The dispersion of any one of Claims 1 to 4, wherein the molar ratio of the repeating units derived from the hydrophilic non-ionic vinyl monomer and the repeating units derived from the ionic liquid monomer in the copolymer is in the range of from 4:1 to 200:1 and preferably in the range of from 5:1 to 100:1.

6. The dispersion of any one of Claims 1 to 5, wherein the copolymer, which is preferably contained in the dispersion in a concentration of 0.01 to 10 % by weight, is a random copolymer.

7. The dispersion of any one of Claims 1 to 6, wherein the copolymer consists of the repeating units derived from the ionic liquid monomer and the repeating units derived from the hydrophilic non-ionic vinyl monomer.

8. The dispersion of any one of Claims 1 to 7, wherein the copolymer is further **characterized in that** the ionic liquid monomers comprise the following:
(i) a cationic group selected from the group consisting of imidazolium, pyridinium, alkylammonium, alkylphosphonium, pyrrolidinium and guanidinium, preferably an imidazolium group; and/or
(ii) an anion selected from the group consisting of halides, NO₃-, BF₄⁻, [BR¹R²R³R⁴]⁻, PF₆-, [(CF₃SO₂)₂N]⁻, [CF₃SO₃]⁻, [CF₃CO₂]⁻ and [CH₃CO₂]⁻, wherein R¹, R², R³ and R⁴ each represent alkyl or aryl.

9. The dispersion of any one of Claims 1 to 8, wherein the ionic liquid monomer, from which the repeating unit in the copolymer is derived, is represented by the following formula (I): 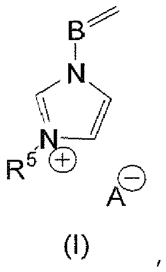 wherein R⁵ is alkyl, aryl or arylalkyl; B represents a linking group and A⁻ represents an anion.

10. The dispersion of Claim 9, wherein in formula (I) R⁵ is C₁₋₆ alkyl and B is CH.

11. The dispersion of any one of Claims 1 to 11, wherein the hydrophilic non-ionic vinyl monomers are selected from the group consisting of acrylamides, acrylates, methacrylates and styrenic monomers.

12. A copolymer comprising repeating units derived from ionic liquid monomer and repeating units derived from hydrophilic non-ionic vinyl monomer, the molar ratio of the repeating units derived from the hydrophilic non-ionic vinyl monomer and the repeating units derived from the ionic liquid monomer being in the range of from 4:1 to 200:1 and preferably in the range of from 5:1 to 100:1 **characterized in that** the hydrophilic non-ionic vinyl monomers are an acrylamide or a styrenic monomer.

13. The copolymer of Claim 12, wherein the ionic liquid monomer is as defined in any one of Claims 8 to 10.

14. A method of dispersing carbon nanomaterial and/or hydrophobic compounds in a dispersion medium, comprising the step of dispersing the carbon nanomaterial and/or hydrophobic compounds in the dispersion medium by introducing an amount of energy sufficient for the dispersion, preferably by ultrasonic treatment, in the presence of a copolymer as defined in any one of Claims 1 or 6 to 15 as a dispersing agent.

15. A use of a copolymer as defined in any one of Claims 1 or 5 to 12 for exfoliating carbon nanostructures, preferably carbon nanotubes or graphene.
